(19) **Europäisches Patentamt**
**European Patent Office**
**Office européen des brevets**

(11) **EP 4 099 000 B1**

(12) **EUROPEAN PATENT SPECIFICATION**

(45) Date of publication and mention
of the grant of the patent:
**24.07.2024 Bulletin 2024/30**

(21) Application number: **22175561.4**

(22) Date of filing: **25.05.2022**

(51) International Patent Classification (IPC):
**G01N 19/02** (2006.01) **G01M 17/02** (2006.01)
**G01N 3/08** (2006.01) **G01N 33/44** (2006.01)

(52) Cooperative Patent Classification (CPC):
**G01N 19/02; G01M 17/02; G01N 3/08;**
**G01N 33/445;** G01N 2203/0075; G01N 2203/0092;
G01N 2203/0216

(54) **METHODS FOR EVALUATING FRICTION PERFORMANCE OF RUBBER COMPOSITION AND MANUFACTURING METHOD FOR TIRE**

VERFAHREN ZUM BEWERTEN DES REIBVERHÄLTNISSES EINER KAUTSCHUK-ZUSAMMENSETZUNG UND HERSTELLUNGSVERFAHREN FÜR REIFEN

PROCÉDÉS D'ÉVALUATION DE PERFORMANCE DE FRICTION D'UNE COMPOSITION DE CAOUTCHOUC ET PROCÉDÉ DE FABRICATION D'UN PNEU

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **04.06.2021 JP 2021094153**

(43) Date of publication of application:
**07.12.2022 Bulletin 2022/49**

(73) Proprietor: **Sumitomo Rubber Industries, Ltd.**
**Kobe-shi, Hyogo-ken 651-0072 (JP)**

(72) Inventor: **MUKAI, Shoichi**
**Kobe-shi, Hyogo-ken, 651-0072 (JP)**

(74) Representative: **Manitz Finsterwald**
**Patent- und Rechtsanwaltspartnerschaft mbB**
**Martin-Greif-Strasse 1**
**80336 München (DE)**

(56) References cited:
**CN-U- 211 904 969     JP-A- 2017 125 816**
**JP-B2- 3 540 088     US-A1- 2014 231 221**

• **GEHMAN S D ET AL: "Mechanics of pneumatic tires NBS MONO 122", 1 November 1971 (1971-11-01), pages 1 - 864, XP061044290, Retrieved from the Internet <URL:https://nvlpubs.nist.gov/nistpubs/Legacy/ MONO/nbsmonograph122.pdf> [retrieved on 19711101], DOI: 10.6028/NBS.MONO.122**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

## Description

BACKGROUND OF THE INVENTION

Field of the Invention

The present invention relates to an evaluation method for a rubber composition. Specifically, the present invention relates to a method for evaluating the friction performance of a rubber composition.

Background Art

A pneumatic tire has a tread. A main material of the tread is a crosslinked rubber composition. The tread comes into direct contact with a road surface. The tread rubs against the road surface. For the tire, the friction performance of a rubber composition of the tread is an important characteristic.

Japanese Laid-Open Patent Publication No. 2017-67453 discloses a method for evaluating the friction performance of a rubber composition by using the viscoelasticity of the rubber composition. In this method, a friction coefficient is predicted on the basis of a loss tangent. In this method, the friction performance can be evaluated without performing a friction test.

The accuracy of evaluation of the evaluation method disclosed in Japanese Laid-Open Patent Publication No. 2017-67453 is insufficient. An object of the present invention is to provide an evaluation method, for a rubber composition, which can be efficiently performed and by which friction performance can be accurately evaluated. The features of the preamble of the independent claims are known from JP 2017 125816 A. Related technologies are known from CN 211 904 969 U, US 2014/231221 A1; JP 3 540088 B2, and GEHMAN S D ET AL: "Mechanics of pneumatic tires NBS MONO 122", NIST, 1 November 1971, pages 1-864, DOI: 10.6028/NBS.MONO.122.

SUMMARY OF THE INVENTION

The present invention is set out in the appended claims. A friction performance evaluation method for a rubber composition according to claim 1 includes the steps of:

(A) calculating a tensile stress or a compressive stress generated in a model, for which an assumed material is a rubber composition, near a projection by simulation when the model slides on a surface having the projection;
(B) determining a reference stress on the basis of the tensile stress or the compressive stress;
(C) obtaining a tensile strain and a compressive strain of the rubber composition when the reference stress is applied to the rubber composition; and
(D) comparing the tensile strain and the compressive strain.

The step (C) includes the steps of:

(C-1) subjecting a first test piece whose material is the rubber composition, to a tensile test to measure the tensile strain; and
(C-2) subjecting a second test piece whose material is the rubber composition, to a compression test to measure the compressive strain. Preferably, in the step (A), the tensile stress or the compressive stress is calculated by a finite element method.

Preferably, the second test piece subjected to the compression test in the step (C-2) has a shape that is the same as that of the first test piece subjected to the tensile test in the step (C-1).

Preferably, a difference or a ratio between the two strains is calculated in the step (D).

Preferably, by the method, a friction coefficient of the rubber composition on a wet road surface is evaluated by comparison with a friction coefficient of the rubber composition on a dry road surface.

According to another aspect, a manufacturing method for a tire according to claim 6 includes the steps of:

(A) calculating a tensile stress or a compressive stress generated in a model, for which an assumed material is a rubber composition, near a projection by simulation when the model slides on a surface having the projection;
(B) determining a reference stress on the basis of the tensile stress or the compressive stress;
(C) obtaining a tensile strain and a compressive strain of the rubber composition when the reference stress is applied to the rubber composition;
(D) comparing the tensile strain and the compressive strain;
(E) determining pass or fail of the rubber composition on the basis of the comparison;
(F) determining specifications of a tread of a tire on the basis of a constitution of the rubber composition that has passed; and
(G) producing the tread having the specifications.

According to still another aspect, a friction performance evaluation method for a rubber composition according to the present invention includes the steps of:

(1) calculating a tensile stress and a compressive stress generated in a model, for which an assumed material is a rubber composition, near a projection by simulation when the model slides on a surface having the projection;
(2) determining a first reference stress on the basis of the tensile stress, and determining a second reference stress on the basis of the compressive stress;

(3) obtaining a tensile strain of the rubber composition when the first reference stress is applied to the rubber composition, and a compressive strain of the rubber composition when the second reference stress is applied to the rubber composition; and
(4) comparing the tensile strain and the compressive strain,

wherein the step (3) includes the steps of:

(3-1) subjecting a first test piece whose material is the rubber composition to a tensile test to measure the tensile strain; and

(3-2) subjecting a second test piece whose material is therubber composition, to a compression test to mesaure the compressive strain.

According to still another aspect, a manufacturing method for a tire according to claim 8 includes the steps of:

(1) calculating a tensile stress and a compressive stress generated in a model, for which an assumed material is a rubber composition, near a projection by simulation when the model slides on a surface having the projection;
(2) determining a first reference stress on the basis of the tensile stress, and determining a second reference stress on the basis of the compressive stress;
(3) obtaining a tensile strain of the rubber composition when the first reference stress is applied to the rubber composition, and a compressive strain of the rubber composition when the second reference stress is applied to the rubber composition;
(4) comparing the tensile strain and the compressive strain;
(5) determining pass or fail of the rubber composition on the basis of the comparison;
(6) determining specifications of a tread of a tire on the basis of a constitution of the rubber composition that has passed; and
(7) producing the tread having the specifications.

By the evaluation method according to the present invention, the friction performance of a rubber composition can be accurately evaluated.

BRIEF DESCRIPTION OF THE DRAWINGS

FIG. 1 is a flowchart showing an evaluation method according to an embodiment of the present invention;
FIG. 2 is a schematic diagram showing a part of a model used in the evaluation method in FIG. 1;
FIG. 3 is a schematic diagram showing the model in FIG. 2 together with a model of a projection;
FIG. 4 is a graph showing stress-strain curves used in the evaluation method in FIG. 1;
FIG. 5 is a graph showing a strain difference-friction coefficient reduction width curve used in the evaluation method in FIG. 1;
FIG. 6 is a graph showing a strain difference-friction coefficient curve used in the evaluation method in FIG. 1;
FIG. 7 is a graph showing stress-strain curves used in an evaluation method according to another embodiment of the present invention; and
FIG. 8 is a flowchart showing a manufacturing method for a tire according to an embodiment of the present invention.

DETAILED DESCRIPTION

Hereinafter, the present invention will be described in detail based on preferred embodiments with appropriate reference to the drawings.

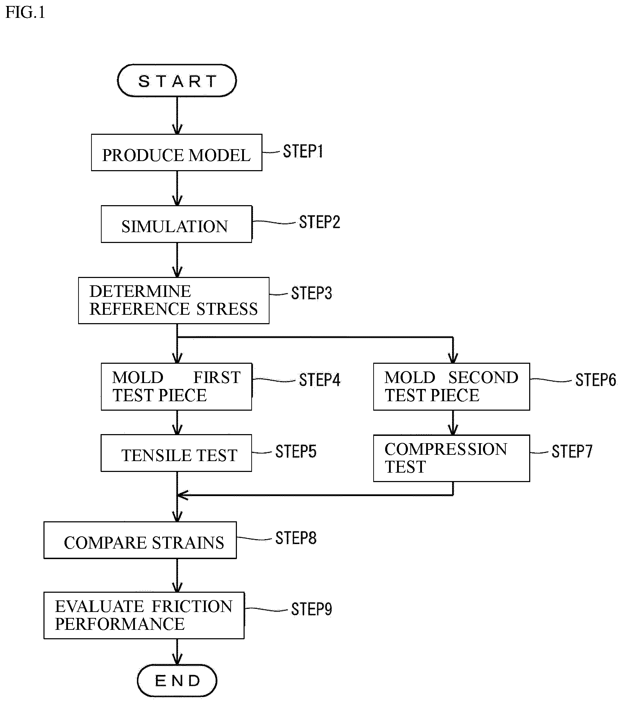

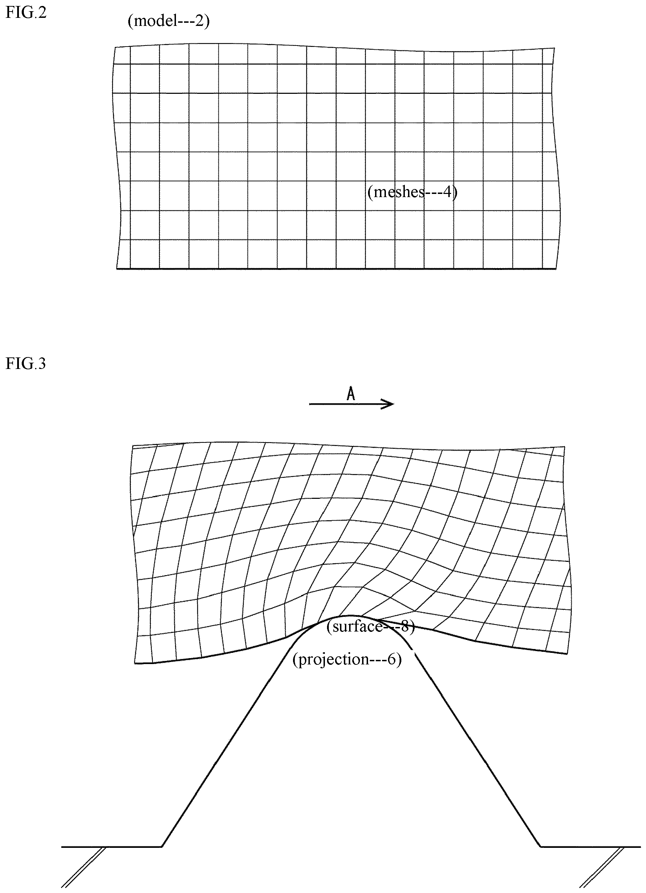

FIG. 1 is a flowchart showing an evaluation method according to an embodiment of the present invention. In the evaluation method, a model is produced (STEP 1). FIG. 2 shows this model 2. The model 2 has a large number of meshes 4. The model 2 does not exist. The model 2 is virtualized. An assumed material of the model 2 is a rubber composition. Therefore, the model 2 has elasticity. A rubber composition for which a detailed constitution thereof such as the type of a base rubber, the types of additives, and the amounts of the additives is specified is assumed as the material. A rubber composition for which detailed characteristics such as hardness are specified is assumed as the material.
The model 2 is subjected to simulation (STEP 2). A typical method of the simulation is a finite element method (FEM). FIG. 3 shows a state of analysis by the finite element method. FIG. 3 also shows a model of a surface 8 having a projection 6 together with the model 2 shown in FIG. 2. Although not shown, the surface 8 is also divided into a large number of meshes. In the present embodiment, the projection 6 is a rigid body. The size of the model 2 of the rubber composition is sufficiently larger than the size of the projection 6.
In the simulation, the model 2 moves in the direction of an arrow A in FIG. 3. The model 2 moves while sliding on the surface 8. Due to this movement, tensile stress is generated in the model 2 on the front side (right side in FIG. 3) of the projection 6. The tensile stress of each mesh 4 is calculated by simulation. These tensile stresses have a distribution. The tensile stresses of the meshes 4 close to the projection 6 are large. A representative value Vt is determined from these tensile stresses. The average value of a large number of tensile stresses may be the representative value Vt. The maximum value of a number of tensile stresses may be the representative value Vt. The representative value Vt may be determined by other means.

Due to the movement of the model 2, compressive stress is generated in the model 2 on the back side (left side in FIG. 3) of the projection 6. The compressive stress of each mesh 4 is calculated by simulation. These compressive stresses have a distribution. The compressive stresses of the meshes 4 close to the projection 6 are large. A representative value Vc is determined from these compressive stresses. The average value of a large number of compressive stresses may be the representative value Vc. The maximum value of a large number of compressive stresses may be the representative value Vc. The representative value Vc may be determined by other means.

Examples of software suitable for the simulation include trade name "LS-DYNA" of Livermore Software Technology and trade name "Abaqus" of Dassault Systemes.

A reference stress Rs is determined on the basis of the representative value Vt of the tensile stress and the representative value Vc of the compressive stress obtained by the simulation (STEP 3). The average of the representative value Vt and the representative value Vc may be the reference stress Rs. The larger one out of the representative value Vt and the representative value Vc may be the reference stress Rs. The smaller one out of the representative value Vt and the representative value Vc may be the reference stress Rs. The reference stress Rs may be determined by other means.

The reference stress Rs may be determined on the basis of the representative value Vt without considering the representative value Vc. In this case, it is not necessary to calculate the compressive stress in the simulation (STEP 2).

The reference stress Rs may be determined on the basis of the representative value Vc without considering the representative value Vt. In this case, it is not necessary to calculate the tensile stress in the simulation (STEP 2).

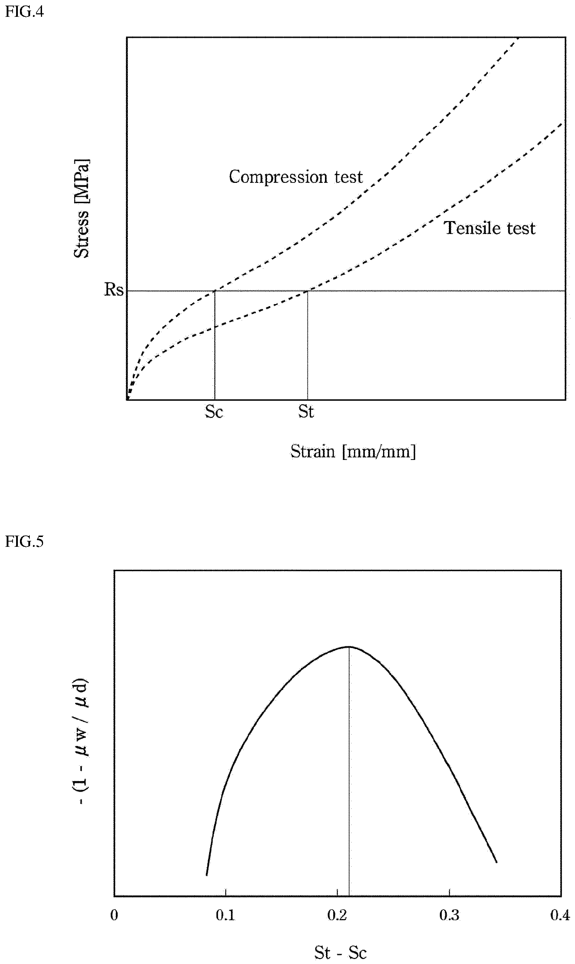

Next, a first test piece is molded (STEP 4). The material of the first test piece is the rubber composition assumed for the model 2 used in the simulation. The first test piece is subjected to a tensile test (STEP 5). The tensile test is performed by a known method. An example of the method of the tensile test is specified in "JIS K 6251". An example of an apparatus suitable for the tensile test is trade name "Autograph" of SHIMADZU CORPORATION. A stress-strain curve is obtained by the tensile test. FIG. 4 shows an example of the stress-strain curve. In FIG. 4, reference character St denotes the tensile strain when the reference stress Rs is applied to the first test piece.

In the method, the first test piece is molded, and the tensile strain St is measured. The tensile strain St may be calculated by simulation.

Next, a second test piece is molded (STEP 6). The material of the second test piece is the same as the material of the first test piece. In other words, the material of the second test piece is the rubber composition assumed for the model 2 used in the simulation. Preferably, the shape of the second test piece is the same as the shape of the first test piece. The second test piece is subjected to a compression test (STEP 7). The compression test is performed by a known method. An example of the method of the compression test is specified in JIS K 6254. An example of an apparatus suitable for the compression test is the above-described trade name "Autograph" of SHIMADZU CORPORATION. A stress-strain curve is obtained by the compression test. FIG. 4 shows an example of the stress-strain curve. In FIG. 4, reference character Sc denotes the compressive strain when the reference stress Rs is applied to the second test piece.

In the method, the second test piece is molded, and the compressive strain Sc is measured. The compressive strain Sc may be calculated by simulation.

Prior to the tensile test (STEP 5), the compression test (STEP 7) may be performed. The tensile test (STEP 5) and the compression test (STEP 7) may be performed in parallel.

The tensile strain St and the compressive strain Sc are compared (STEP 8). In the present embodiment, a difference (St - Sc) is calculated as this comparison. The friction performance is evaluated on the basis of the difference (St - Sc) (STEP 9). The difference (St - Sc) is one type of strain parameter.

FIG. 5 shows a graph used in the evaluation. The graph is prepared prior to performing the evaluation method according to the present invention. The graph is obtained by measuring the friction coefficients of a large number of rubber compositions. In the graph, the horizontal axis indicates the difference (St - Sc). In the graph, the vertical axis indicates an index Id representing the amount of decrease from a friction coefficient $\mu d$ on a dry road surface to a friction coefficient $\mu w$ on a wet road surface. The index Id is calculated by the following equation.

$$ Id = -(1 - \mu w / \mu d) $$

This Id is one type of friction coefficient parameter. The friction coefficient $\mu w$ on a wet road surface which is compared with the friction coefficient $\mu d$ on a dry road surface can be evaluated on the basis of the index Id.

For a general rubber composition, a friction coefficient $\mu w$ on a wet road surface is lower than a friction coefficient $\mu d$ on a dry road surface. For a rubber composition for which the index Id is high (close to zero), the difference between the friction coefficient $\mu w$ and the friction coefficient $\mu d$ is small. In other words, the dependence of the friction performance of the rubber composition on the state of a road surface is small.

As shown in FIG. 5, for a rubber composition having a difference (St - Sc) of about 0.21, the index Id is high. In other words, for a rubber composition having a difference (St - Sc) of 0.21 or close to 0.21, the dependence of the friction performance on the state of a road surface is small. In the present embodiment, a rubber composition for which the difference (St - Sc) is 0.21 or close to 0.21 is evaluated to "have excellent friction performance". A rubber composition having a difference (St - Sc) significantly smaller than 0.21 is evaluated to "have inferior friction performance". A rubber composition having a difference (St - Sc) significantly larger than 0.21 is evaluated to "have inferior friction performance". In an evaluation method in which a graph different from FIG. 5 is used, a preferable difference (St - Sc) can be a value other than 0.21.

In an actual slip, the rubber composition repeats tensile deformation and restoration. The rubber composition further repeats compressive deformation and restoration. The behavior of the slip is complex. The present inventor has found that the friction performance of a rubber composition can be accurately determined by comparing tensile strain and compressive strain. This evaluation method does not require real measurement of the friction coefficient of the rubber composition. The evaluation method is simple.

As the horizontal axis of the graph in FIG. 5, another value may be set. In other words, the friction performance may be evaluated on the basis of another strain parameter. Examples of the other strain parameter include a difference (Sc - St), a ratio (St / Sc), and a ratio (Sc / St). The value obtained by substituting the strength strain St and the compressive strain Sc into a predetermined relational expression may be used as the strain parameter.

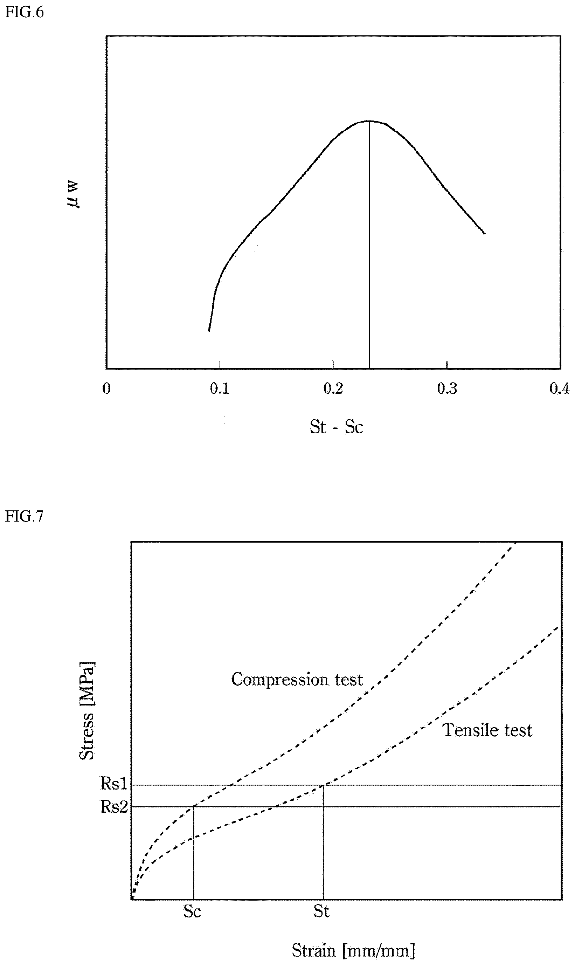

The friction performance may be evaluated on the basis of a friction coefficient parameter other than the index Id. An example of this parameter is the friction coefficient $\mu w$ on a wet road surface. In a graph shown in FIG. 6, the horizontal axis indicates the difference (St - Sc), and the vertical axis indicates the friction coefficient $\mu w$ on a wet road surface. As shown in FIG. 6, for a rubber composition having a difference (St - Sc) of about 0.23, the friction coefficient $\mu w$ is high. In the present embodiment, a rubber composition having a difference (St - Sc) of 0.23 or close to 0.23 is evaluated to "have excellent friction performance on a wet road surface". A rubber composition having a difference (St - Sc) significantly smaller than 0.23 is evaluated to "have inferior friction performance on a wet road surface". A rubber composition having a difference (St - Sc) significantly larger than 0.23 is evaluated to "have inferior friction performance on a wet road surface".

The friction coefficient parameter may be the friction coefficient $\mu d$ on a dry road surface. The friction coefficient parameter may be a friction coefficient on a snow or ice road surface. The friction coefficient parameter may be a relational expression between the friction coefficient on a snow or ice road surface and the friction coefficient $\mu d$ on a dry road surface.

The friction performance may be evaluated without using any graph. In this case, whether the friction performance is good or bad is directly determined by comparing the tensile strain St and the compressive strain Sc. For example, a rubber composition having a difference (St - Sc) of 0.21 or close to 0.21 is evaluated to "have excellent friction performance".

FIG. 7 is a graph showing stress-strain curves used in an evaluation method according to another embodiment of the present invention. In the evaluation method shown in FIG. 1, both the tensile stress and the compressive stress are taken into consideration in determining the reference stress Rs (STEP 3). On the other hand, in the evaluation method shown in FIG. 7, a first reference stress Rs1 is determined on the basis of tensile stress, and a second reference stress Rs2 is determined on the basis of compressive stress. On the basis of the stress-strain curves shown in FIG. 7, the tensile strain St corresponding to the first reference stress Rs1 is measured, and the compressive strain Sc corresponding to the second reference stress Rs2 is measured. The friction performance is evaluated by comparing the tensile strain St and the compressive strain Sc. A specific evaluation method is the same as the method in the embodiment shown in FIGS. 1 to 6. The tensile strain St may be calculated by simulation in which the first reference stress Rs1 is used. The compressive strain Sc may be calculated by simulation in which the second reference stress Rs2 is used.

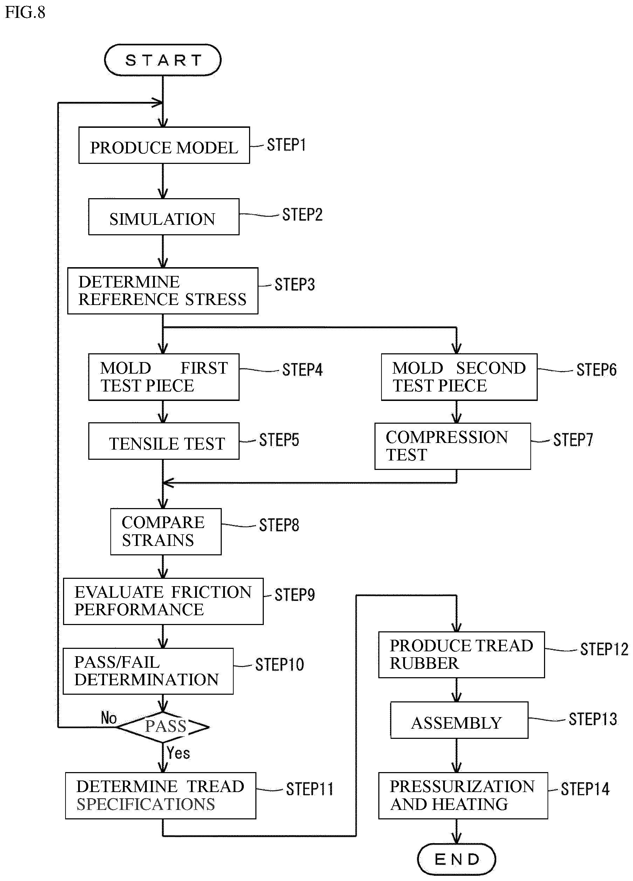

FIG. 8 is a flowchart showing a manufacturing method for a tire according to an embodiment of the present invention. In the manufacturing method, in the same manner as the evaluation method shown in FIG. 1, production of a model 2 (STEP 1), simulation (STEP 2), determination of reference stresses (STEP 3), molding of a first test piece (STEP 4), a tensile test (STEP 5), molding of a second test piece (STEP 6), a compression test (STEP 7), comparison of strains (STEP 8), and evaluation of friction performance (STEP 9) are performed. The evaluation of friction performance (STEP 9) may be omitted.

Furthermore, in the manufacturing method, determination as to whether a rubber composition passes or fails (STEP 10) is performed. In this step, it is determined whether a difference (St - Sc) is within a predetermined range. If the rubber composition does not pass in this determination, a model 2 of another rubber composition having a different constitution is produced (STEP 1). Steps from the simulation (STEP 2) to the pass/fail determination (STEP 10) for this rubber composition are performed. A rubber composition having excellent friction performance is found by repeating these steps.

If the rubber composition passes in the determination (STEP 10), the specifications of a tread are determined (STEP 11). Specifically, the constitution of a rubber composition for a tread is determined. The constitution of the rubber composition for a tread may be the same as the constitution of the rubber composition that has passed in the determination (STEP 10). The constitution of the rubber composition for a tread may be different from the constitution of the rubber composition that has passed in the determination (STEP 10). In other words, the constitution of the rubber composition for a tread can be determined by referring to the constitution of the rubber composition that has passed in the determination (STEP 10). The constitution of the rubber composition for a tread can be determined so as to have even better friction performance than the friction performance of the rubber composition that has passed in the determination (STEP 10).

A tread rubber is produced according to the determined specifications (STEP 12). The tread rubber is assembled with other rubber members (STEP 13). As a result of the assembly, a raw cover (green tire) is obtained. The raw cover is placed into a mold, and pressurized and heated (STEP 14). Due to the pressurization and the heating, the rubber composition flows in the cavity of the mold. Due to the heating, the rubber molecules of the rubber composition cause a crosslinking reaction. Accordingly, a tire is obtained. The tire has excellent friction performance. By the manufacturing method, a tire having excellent friction performance is easily obtained.

In the manufacturing method, a first test piece is molded, and a tensile strain St is measured (STEP 4, STEP 5). The tensile strain St may be calculated by simulation. In the manufacturing method, a second test piece is molded, and a compressive strain Sc is measured (STEP 6, STEP 7). The compressive strain Sc may be calculated by simulation.

In the manufacturing method, the pass/fail determination (STEP 10) may be performed on the basis of a strain parameter other than the difference (St - Sc). For example, the pass/fail determination can be performed on the basis of a difference (Sc - St), a ratio (St / Sc), or a ratio (Sc / St). The pass/fail determination may be performed on the basis of the value obtained by substituting the tensile strain St and the compressive strain Sc into a predetermined relational expression. The pass/fail determination (STEP 10) may be performed on the basis of a friction coefficient parameter.

In the manufacturing method, a first reference stress Rs1 may be determined on the basis of tensile stress, and a second reference stress Rs2 may be determined on the basis of compressive stress. In this case, the tensile strain St corresponding to the first reference stress Rs1 is measured on the basis of a stress-strain curve. The compressive strain Sc corresponding to the second reference stress Rs2 is measured on the basis of a stress-strain curve. The pass/fail determination (STEP 10) can be performed by comparing the tensile strain St and the compressive strain Sc.

The evaluation method described above is suitable for evaluating the friction performance of various rubber products such as soles, floors, and conveyors.

EXPLANATION OF NUMERALS

2     Model

4     Meshes

6     Projection

8     Surface

## Claims

1.  A friction performance evaluation method for a rubber composition, **characterized by** comprising the steps of:

    (A) calculating a tensile stress or a compressive stress generated in a model (2), for which an assumed material is a rubber composition, near a projection (6) by simulation when the model (2) slides on a surface (8) having the projection (6);
    (B) determining a reference stress (Rs) on the basis of the tensile stress or the compressive stress;
    (C) obtaining a tensile strain (St) and a compressive strain (Sc) of the rubber composition when the reference stress (Rs) is applied to the rubber composition; and
    (D) comparing the tensile strain (St) and the compressive strain (Sc);

    wherein the step (C) includes the steps of:

    (C-1) subjecting a first test piece whose material is the rubber composition, to a tensile test to measure the tensile strain (St); and
    (C-2) subjecting a second test piece whose material is the rubber composition, to a compression test to measure the compressive strain (Sc).

2.  The evaluation method according to claim 1, wherein, in the step (A), the tensile stress or the compressive stress is calculated by a finite element method.

3.  The evaluation method according to claim 1 or 2, wherein the second test piece subjected to the compression test in the step (C-2) has a shape that is the same as that of the first test piece subjected to the tensile test in the step (C-1).

4.  The evaluation method according to any one of claims 1 to 3, wherein a difference (St - Sc, Sc - St) or a ratio (St / Sc, Sc / St) between the two strains (St, Sc) is calculated in the step (D).

5.  The evaluation method according to any one of claims 1 to 4, wherein a friction coefficient (μw) of

the rubber composition on a wet road surface is evaluated by comparison with a friction coefficient (μd) of the rubber composition on a dry road surface.

**6.** A manufacturing method for a tire, comprising the method of any one of claims 1 to 5 and further comprising the steps of:

(E) determining pass or fail of the rubber composition on the basis of the comparison;
(F) determining specifications of a tread of a tire on the basis of a constitution of the rubber composition that has passed; and
(G) producing the tread having the specifications.

**7.** A friction performance evaluation method for a rubber composition, **characterized by** comprising the steps of:

(1) calculating a tensile stress and a compressive stress generated in a model (2), for which an assumed material is a rubber composition, near a projection (6) by simulation when the model (2) slides on a surface (8) having the projection (6);
(2) determining a first reference stress (Rs1) on the basis of the tensile stress, and determining a second reference stress (Rs2) on the basis of the compressive stress;
(3) obtaining a tensile strain (St) of the rubber composition when the first reference stress (Rs1) is applied to the rubber composition, and a compressive strain (Sc) of the rubber composition when the second reference stress (Rs2) is applied to the rubber composition; and
(4) comparing the tensile strain (St) and the compressive strain (Sc);

wherein the step (3) includes the steps of:

(3-1) subjecting a first test piece whose material is the rubber composition, to a tensile test to measure the tensile strain (St); and
(3-2) subjecting a second test piece whose material is the rubber composition, to a compression test to measure the compressive strain (Sc).

**8.** A manufacturing method for a tire, comprising the method of claim 7 and further comprising the steps of:

(5) determining pass or fail of the rubber composition on the basis of the comparison;
(6) determining specifications of a tread of a tire on the basis of a constitution of the rubber composition that has passed; and

(7) producing the tread having the specifications.

**Patentansprüche**

**1.** Reibungsleistungs-Bewertungsverfahren für eine Kautschukzusammensetzung, **dadurch gekennzeichnet, dass** es die Schritte umfasst:

(A) Berechnen einer Zugspannung oder einer Druckspannung, die in einem Modell (2) erzeugt wird, für das ein angenommenes Material eine Kautschukzusammensetzung ist, in der Nähe eines Vorsprungs (6) durch Simulation, wenn das Modell (2) auf einer Oberfläche (8), die den Vorsprung (6) aufweist, gleitet;
(B) Bestimmen einer Referenzspannung (Rs) auf der Grundlage der Zugspannung oder der Druckspannung;
(C) Erhalten einer Zugdehnung (St) und einer Druckdehnung (Sc) der Kautschukzusammensetzung, wenn die Referenzspannung (Rs) auf die Kautschukzusammensetzung ausgeübt wird; und
(D) Vergleichen der Zugdehnung (St) und der Druckdehnung (Sc);

wobei der Schritt (C) die Schritte umfasst:

(C-1) Unterziehen eines ersten Probestücks, dessen Material die Kautschukzusammensetzung ist, einem Zugversuch, um die Zugdehnung (St) zu messen; und
(C-2) Unterziehen eines zweiten Probestücks, dessen Material die Kautschukzusammensetzung ist, einem Druckversuch, um die Druckdehnung (Sc) zu messen.

**2.** Bewertungsverfahren nach Anspruch 1, wobei in dem Schritt (A) die Zugspannung oder die Druckspannung durch ein Finite-Elemente-Verfahren berechnet wird.

**3.** Bewertungsverfahren nach Anspruch 1 oder 2, wobei das zweite Probestück, das dem Druckversuch in dem Schritt (C-2) unterzogen wurde, eine Form aufweist, die die gleiche ist wie die des ersten Probestücks, das dem Zugversuch in dem Schritt (C-1) unterzogen wurde.

**4.** Bewertungsverfahren nach einem der Ansprüche 1 bis 3, wobei eine Differenz (St - Sc, Sc - St) oder ein Verhältnis (St / Sc, Sc / St) zwischen den zwei Dehnungen (St, Sc) in dem Schritt (D) berechnet wird.

**5.** Bewertungsverfahren nach einem der Ansprüche 1 bis 4, wobei ein Reibungskoeffizient (μw) der Kaut-

schukzusammensetzung auf einer nassen Straßenoberfläche durch Vergleich mit einem Reibungskoeffizienten ($\mu$d) der Kautschukzusammensetzung auf einer trockenen Straßenoberfläche bewertet wird.

6. Herstellungsverfahren für einen Reifen, das das Verfahren nach einem der Ansprüche 1 bis 5 umfasst und das ferner die Schritte umfasst:

(E) Bestimmen des Bestehens oder Nichtbestehens der Kautschukzusammensetzung auf der Grundlage des Vergleichs;
(F) Bestimmen von Spezifikationen einer Lauffläche eines Reifens auf der Grundlage einer Beschaffenheit der Kautschukzusammensetzung, die bestanden hat; und
(G) Herstellen der Lauffläche mit den Spezifikationen.

7. Reibungsleistungs-Bewertungsverfahren für eine Kautschukzusammensetzung, **dadurch gekennzeichnet, dass** es die Schritte umfasst:

(1) Berechnen einer Zugspannung und einer Druckspannung, die in einem Modell (2) erzeugt werden, für das ein angenommenes Material eine Kautschukzusammensetzung ist, in der Nähe eines Vorsprungs (6) durch Simulation, wenn das Modell (2) auf einer Oberfläche (8), die den Vorsprung (6) aufweist, gleitet;
(2) Bestimmen einer ersten Referenzspannung (Rs1) auf der Grundlage der Zugspannung und Bestimmen einer zweiten Referenzspannung (Rs2) auf der Grundlage der Druckspannung;
(3) Erhalten einer Zugdehnung (St) der Kautschukzusammensetzung, wenn die erste Referenzspannung (Rs1) auf die Kautschukzusammensetzung ausgeübt wird, und einer Druckdehnung (Sc) der Kautschukzusammensetzung, wenn die zweite Referenzspannung (Rs2) auf die Kautschukzusammensetzung ausgeübt wird; und
(4) Vergleichen der Zugdehnung (St) und der Druckdehnung (Sc);

wobei der Schritt (3) die Schritte umfasst:

(3-1) Unterziehen eines ersten Probestücks, dessen Material die Kautschukzusammensetzung ist, einem Zugversuch, um die Zugdehnung (St) zu messen; und
(3-2) Unterziehen eines zweiten Probestücks, dessen Material die Kautschukzusammensetzung ist, einem Druckversuch, um die Druckdehnung (Sc) zu messen.

8. Herstellungsverfahren für einen Reifen, das das Verfahren nach Anspruch 7 umfasst und das ferner die Schritte umfasset:

(5) Bestimmen des Bestehens oder Nichtbestehens der Kautschukzusammensetzung auf der Grundlage des Vergleichs;
(6) Bestimmen von Spezifikationen einer Lauffläche eines Reifens auf der Grundlage einer Beschaffenheit der Kautschukzusammensetzung, die bestanden hat; und
(7) Herstellen der Lauffläche mit den Spezifikationen.

**Revendications**

1. Procédé d'évaluation de performance de friction pour une composition de caoutchouc, **caractérisé en ce qu'**il comprend les étapes consistant à :

(A) calculer une contrainte de traction ou une contrainte de compression générée dans un modèle (2), pour lequel un matériau présumé est une composition de caoutchouc, près d'une projection (6) par simulation quand le modèle (2) glisse sur une surface (8) ayant la projection (6) ;
(B) déterminer une contrainte de référence (Rs) sur la base de la contrainte de traction ou de la contrainte de compression ;
(C) obtenir une contrainte de traction (St) et une contrainte de compression (Sc) de la composition de caoutchouc quand la contrainte de référence (Rs) est appliquée sur la composition de caoutchouc ; et
(D) comparer la contrainte de traction (St) et la contrainte de compression (Sc) ;

dans lequel l'étape (C) inclut les étapes consistant à :

(C-1) soumettre une première pièce d'essai, dont le matériau est la composition de caoutchouc, à un essai de traction pour mesurer la contrainte de traction (St) ; et
(C-2) soumettre une seconde pièce d'essai, dont le matériau est la composition de caoutchouc, à un essai de compression pour mesurer la contrainte de compression (Sc).

2. Procédé d'évaluation selon la revendication 1, dans lequel, dans l'étape (A), la contrainte de traction ou la contrainte de compression est calculée par le biais d'une méthode des éléments finis.

3. Procédé d'évaluation selon la revendication 1 ou 2, dans lequel la seconde pièce d'essai soumise à l'essai de compression dans l'étape (C-2) a une forme qui est la même que celle de la première pièce d'essai soumise à l'essai de traction dans l'étape (C-1).

4. Procédé d'évaluation selon l'une quelconque des revendications 1 à 3, dans lequel une différence (St - Sc, Sc - St) ou un rapport (St / Sc, Sc / St) entre les deux contraintes (St, Sc) est calculé(e) dans l'étape (D).

5. Procédé d'évaluation selon l'une quelconque des revendications 1 à 4, dans lequel un coefficient de friction ($\mu$w) de la composition de caoutchouc sur une surface routière mouillée est évalué par comparaison à un coefficient de friction ($\mu$d) de la composition de caoutchouc sur une surface routière sèche.

6. Procédé de fabrication pour un pneumatique, comprenant le procédé selon l'une quelconque des revendications 1 à 5 et comprenant en outre les étapes consistant à :

   (E) déterminer une réussite ou un échec de la composition de caoutchouc sur la base de la comparaison ;
   (F) déterminer des spécifications d'une bande de roulement d'un pneumatique sur la base d'une constitution de la composition de caoutchouc qui a réussi ; et
   (G) produire la bande de roulement ayant les spécifications.

7. Procédé d'évaluation de performance de friction pour une composition de caoutchouc, **caractérisé en ce qu'**il comprend les étapes consistant à :

   (1) calculer une contrainte de traction et une contrainte de compression générée dans un modèle (2), pour lequel un matériau présumé est une composition de caoutchouc, près d'une projection (6) par simulation quand le modèle (2) glisse sur une surface (8) ayant la projection (6) ;
   (2) déterminer une première contrainte de référence (Rs1) sur la base de la contrainte de traction, et déterminer une seconde contrainte de référence (Rs2) sur la base de la contrainte de compression ;
   (3) obtenir une contrainte de traction (St) de la composition de caoutchouc quand la première tension de référence (Rs1) est appliquée sur la composition de caoutchouc, et une contrainte de compression (Sc) de la composition de caoutchouc quand la seconde contrainte de référence (Rs2) est appliquée sur la composition de caoutchouc ; et
   (4) comparer la contrainte de traction (St) et la contrainte de compression (Sc) ;

   dans lequel l'étape (3) inclut les étapes consistant à :

   (3-1) soumettre une première pièce d'essai, dont le matériau est la composition de caoutchouc, à un essai de traction pour mesurer la contrainte de traction (St) ; et
   (3-2) soumettre une seconde pièce d'essai, dont le matériau est la composition de caoutchouc, à un essai de compression pour mesurer la contrainte de compression (Sc).

8. Procédé de fabrication pour un pneumatique, comprenant le procédé selon la revendication 7 et comprenant en outre les étapes consistant à :

   (5) déterminer une réussite ou un échec de la composition de caoutchouc sur la base de la comparaison ;
   (6) déterminer des spécifications d'une bande de roulement d'un pneumatique sur la base d'une constitution de la composition de caoutchouc qui a réussi ; et
   (7) produire la bande de roulement ayant les spécifications.

FIG.1

START

PRODUCE MODEL — STEP1

SIMULATION — STEP2

DETERMINE
REFERENCE STRESS — STEP3

MOLD FIRST
TEST PIECE — STEP4

MOLD SECOND
TEST PIECE — STEP6

TENSILE TEST — STEP5

COMPRESSION
TEST — STEP7

COMPARE STRAINS — STEP8

EVALUATE FRICTION
PERFORMANCE — STEP9

END

FIG.2

(model---2)

(meshes---4)

FIG.3

A

(surface---8)

(projection---6)

FIG.4

FIG.5

FIG.6

FIG.7

FIG.8

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2017067453 A
- JP 2017125816 A
- CN 211904969 U
- US 2014231221 A1
- JP 3540088 B

**Non-patent literature cited in the description**

- **GEHMAN S D et al.** Mechanics of pneumatic tires NBS MONO 122. *NIST,* 01 November 1971, 1-864